(19) Europäisches Patentamt
European Patent Office
Office européen des brevets

(11) **EP 2 019 659 B1**

(12) **EUROPEAN PATENT SPECIFICATION**

(45) Date of publication and mention
of the grant of the patent:
**20.04.2016 Bulletin 2016/16**

(21) Application number: **07718814.2**

(22) Date of filing: **02.05.2007**

(51) Int Cl.:
***A61F 13/42*** *(2006.01)*

(86) International application number:
**PCT/AU2007/000567**

(87) International publication number:
**WO 2007/128038 (15.11.2007 Gazette 2007/46)**

(54) **MOISTURE MONITORING SYSTEM**

FEUCHTIGKEITSÜBERWACHUNGSSYSTEM

SYSTÈME DE SURVEILLANCE D'HUMIDITÉ

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **02.05.2006 AU 2006902251**

(43) Date of publication of application:
**04.02.2009 Bulletin 2009/06**

(73) Proprietor: **Fred Bergman Healthcare Pty Ltd North Sidney, NSW 2060 (AU)**

(72) Inventors:
• **BARDA, David, Albert**
**Docklands, Victoria 3008 (AU)**
• **WEINSTOCK, Daniel**
**Caulfield South, Victoria 3162 (AU)**
• **GUIBERT, Remi**
**Mount Martha, Victoria 3934 (AU)**

• **RODDA, Maria, C**
**Mount Eliza, Victoria 3150 (AU)**
• **EITZEN, Guy**
**Wheelers Hill, Victoria 3150 (AU)**

(74) Representative: **Tranter, Andrew David et al Barker Brettell LLP**
**100 Hagley Road**
**Edgbaston**
**Birmingham B16 8QQ (GB)**

(56) References cited:
WO-A1-2005/092177    WO-A2-2004/100763
WO-A2-2004/100763    CA-A1- 2 361 132
CA-A1- 2 361 132    JP-A- 09 206 292
JP-A- 2001 318 067    JP-A- 2002 301 098
US-A- 5 416 469    US-A- 5 568 128
US-A1- 2003 011 479    US-A1- 2003 011 479
US-A1- 2004 220 538    US-A1- 2004 220 538
US-A1- 2005 046 578

**Description**

Field of the Invention

The present invention relates to moisture monitoring. It relates particularly but not exclusively to systems, devices and methods for monitoring moisture in absorbent articles such as diapers, incontinence garments, dressings and pads, resulting from wetness events caused by, for example, urinary and/or faecal incontinence.

Background to the Invention

Incontinence is a condition in which there is uncontrolled release of natural discharges or evacuations. While some forms of incontinence are more widespread, the condition usually affects women, the elderly and the infirm. Urinary incontinence refers to loss of bladder control resulting in involuntary or uncontrolled urination. Other forms of incontinence including faecal or bowel incontinence also exist. In the context of the present application, the term "incontinence" is to be taken to include urinary and faecal incontinence.

A range of different incontinence types are recognised. Stress incontinence refers to involuntary loss of urine immediately associated with coughing, sneezing, lifting, straining or other physical exertion. The term "stress" relates to the mechanical stress of the abdominal muscles compressing the bladder wall, working against weakened sphincter muscles. Childbirth, obesity, constipation and changes in the sphincter muscles after the menopause can aggravate stress incontinence as can the use of some drugs.

Urge incontinence refers to the involuntary loss of urine coupled with a strong desire to urinate. Often the sufferer is unable to reach the toilet before there has been a urine loss. The need to visit the toilet may occur very frequently during the day and often at night also. Urge incontinence is generally caused by an overactive or "unstable" bladder which contracts involuntarily in an attempt to empty. The contractions give rise to an urgent desire to pass urine and uncontrolled leakage occurs before a toilet is reached. Mixed Urinary Incontinence (MUI) refers to involuntary leakage associated with urge incontinence and also with exertion, effort, sneezing, or coughing associated with stress incontinence.

Overflow incontinence refers to involuntary loss of urine associated with a chronically distended and overfull bladder. The bladder may be distended as a result of incomplete emptying which may be caused by obstruction to the outlet of the bladder or as a result of a failure of the bladder muscle to contract properly. Bladder failure of this kind may be caused by disease of the nervous system, by some drugs or by psychological factors.

Dribble incontinence refers to leakage of urine without warning or provocation. This is a demoralising condition because leakage can occur at anytime and is unpredictable. Persons suffering from dribble incontinence often need to wear protective pads or diapers throughout the day and night. Total incontinence is a term sometimes used to describe continuous leaking of urine, day and night, or periodic large volumes of urine and uncontrollable leaking. Some people have this type of incontinence because they were born with an anatomical defect. It can also be caused by a spinal cord injury or by injury to the urinary system from surgery.

Functional incontinence occurs where the ability to get to the toilet is impaired either by physical conditions such as paralysis or arthritis, or mental impairment. This is very common in nursing home patients who rely on assistance from others for mobility.

Although incontinence is relatively widespread, it is a condition which must be treated with sensitivity as it can be uncomfortable and embarrassing for sufferers and carers alike. When left unchecked, incontinence can become more embarrassing due to the existence of unpleasant odours associated with incontinence events and this can create an unpleasant environment for others in the vicinity of the incontinence sufferer. In addition, exudate escaping the body as the result of an incontinence event often contains bacteria, so unchecked wetness can create health and hygiene problems. Also, health regulations or protocols may prescribe a maximum period, for example 15 minutes, for which a patient suffering incontinence may be left in a wet state.

In the past, to comply with regulations and protocols and to ensure that patients in care institutions such as hospitals, nursing homes, aged care facilities and geriatric institutions are well looked after, it has been necessary for staff to manually check patients suffering from incontinence on a regular basis. Apart from the unpleasantness involved with manual checks, such a regimen also places a strain on staff resources. Often manually checking for wetness will also cause interruption to a patient's rest and sleep.

Incontinence indicators and detection systems exist. However, they have done little to improve the current situation in which carers must manually and regularly check patients for wetness. Existing incontinence detection systems are generally unable to distinguish a urinary incontinence event from a fecal incontinence event or the size of these events. Existing systems are also deficient in that they alarm or alert a carer simply when wetness is detected, with no indication of the degree of wetness present. This can cause more time wasted than saved as very small volumes e.g. of urine or perspiration may trigger an alert even though the patient does not actually require attention from a carer. This can also be a source of embarrassment for the patient.

Some systems involve complicated circuitry and are expensive and difficult to manufacture. Since most diapers and pads are disposable both for efficiency of use and hygiene reasons, complicated sensor systems do not lend themselves to widespread uptake and ongoing use.

Some systems are clumsy to use and the sensors can interfere with the absorbent capacity of the diaper or pad with which they are used. Others again are generally incompatible with current care practices and actually create additional work, significant complications or changes in care practices undermining any benefits they may offer and making them less susceptible to widespread uptake and ongoing use.

The present invention aims to improve upon these systems, to improve efficiency in monitoring and management of continence with minimal changes in care practices, or at least provide a useful alternative to existing systems.

WO 2004 100763 discloses a patient monitoring system including a real-time wetness sensor configured to detect the rate of change in wetness occurring within a diaper and automatically adjusting the sensitivity of the sensor to account for a wetness event unrelated to urination. Data associated with detected wetness events is generated and a wireless transmitter sends the generated data through a host computer to a care giver unit. The caregiver can then check the status of the patient, provide a service and annotate the patient's history by transmitting the recorded observation and service provided back to the base station. This system improves quality of care and degree of accountability of caregivers by integrating them into the monitoring-information system.

US2004220538 discloses a hygienic diaper or sensor pad with an alert. The sensors detect diseases, ketone levels, sugar levels, blood, body fluids, liquids, gasses, drugs, bacteria, viruses, toxins or other agents. A powered circuit card unit and transmission circuitry connected to the diaper and sensors within the diaper alert caregivers when the diaper becomes wet or full. Algorithms identify what the sensors detect, and report it to a nurse remote wand or by wired/wireless receiver links to a computer database which can be integrated with a software database to enable staff to input data relating to treatment plans, diagnoses, notes and to track other patient data.

CA2361132 discloses a wetness monitoring system that includes a data collection device that sends wetness measurement data to a central computer that detects changes in wetness caused by the presence of urine or other dielectric fluids. The device includes a semi-reusable sensor and reusable data collector, worn on a garment of the patient. The data collector uses changing resistance characteristics in the sensor to gather wetness data which is periodically generated and transmitted to a central computer. For power conservation, the signals are transmitted less frequently when the sensor is clearly dry, and more frequently when the sensor is damp or a wetness event may have occurred. The Central computer compares the data to an adjustable wetness sensitivity level to determine if a wetness event has occurred. When the central computer determines that a wetness event has occurred, the person is identified and a healthcare worker is paged to attend to that individual.

US2003011479 discloses a sensor device and an analysing device adapted to be used in conjunction with diapers for an incontinent patient. The system is adapted to transmit sensed data to a remote analysing system. The remote analysing system is adapted to manipulate the information and extract various types of valuable data obtained from the sensor, which is adapted to sense both variations in moisture and in temperature as well as other physiological data (e.g. glucose level in the urine or other biochemical markers). The data may indicate e.g. the time period between soiling of the diaper and changing of the diaper by personnel, and may be used to group patients according to their diaper changing needs. The sensed biological data such as temperature, loss of weight or the biochemical contents of exudate can also be used to monitor the wellbeing of the patient.

US 5568128 discloses a self-learning diaper wetness detector for use in infant monitoring. The invention monitors the diaper for dryness by sensing the conductance between two terminals in contact with the diaper. The device aids in toilet training the infant by providing early warning of a likely wetness event, thereby promoting efficient use of diapers and reducing cost of consumption. The learning algorithm uses a twenty-four hour clock to time wetting events. Both event time and probability are updated each day. If a wetting happens at or near the event time, the event probability is incremented and the event time is modified. If wetting does not occur, the event probability is decremented. If the event probability is higher than a set value, the event is tagged as a probable event and a warning alarm can be generated a few minutes before reaching a probable event so that the child can be taken to the toilet.

Other documents on wetness indicators are US5416469, WO2005092177, JP2001318067, JP2002301098.

## Summary of the Invention

According to a first aspect of the present invention, there is provided a moisture monitoring system for monitoring wetness in one or more absorbent articles. The system includes an input for receiving one or more sensor signals indicative of a presence of wetness in an absorbent article, a processor for processing the one or more sensor signals, and a user interface for communicating with a user of the system, characterised in that the processor is adapted to execute an algorithm for performing an analysis of the signals, whereby the algorithm applies the received sensor signals to a pre-determined mathematical model to characterise a wetness event in an absorbent article and to determine a range of predictions based on patterns identified from the sensor signals , and characterised in that parameters of the mathematical model are determined over a training period by the processor (i) continually receiving sensor signals indicating wetness and upon every variation in sensor values, receiving observation data corresponding to (a) changing the pad, (b) examining the pad, and (c) weighing the pad; (ii) performing a regression analysis to formulate parameters for the mathematical model; (iii) feeding the parameters back into the mathematical model to determine a confidence level indicating how accurately the mathematical model estimates the actual events defined by the observation data; and (iv) repeating steps (i) to (iii) until an acceptable confidence level is reached.

The processor may execute an algorithm to devise automatically a mathematical model for characterising a wetness event in an absorbent article. Alternatively, the processor executes an algorithm to perform the analysis, where the algorithm applies the sensor signals to a pre-determined mathematical model to characterise a wetness event in an absorbent article by determining e.g. an estimated volume of exudate in a wetness event and/or the nature of exudate in a wetness event. Alternatively the algorithm may apply variables derived from the one or more sensor signals to the mathematical model.

The processor may apply sensor signals and/or derive variables from the sensor signals to determine one or more parameters suitable for use in a mathematical model for characterising a wetness event. The sensor signals may indicate one or more of conductivity of the exudate, temperature of the absorbent article. The sensor pattern includes one or more of elongate sensor elements, sensor elements arranged in a grid and an array of sensor element dots.

In one embodiment, one or more sensor elements extend beyond an edge of the absorbent article, preferably a front edge and includes a connector for connecting the sensor elements to a signal receiver unit easily without significant disturbance to the patient being monitored.

A cover layer may be provided over the sensor elements which also extends beyond an edge of the absorbent article and includes means such as a pouch, pocket or flap for enclosing a signal receiver unit attachable to one or more of the sensor elements. It is preferable that one or more sensor elements are arranged for connection to a signal receiver unit outside the absorbent article.

The signal receiver unit may include storage means for storing sensor signals collected over a period of time. Alternatively or additionally, the signal receiver unit may include means for receiving data relating to a patient's toileting activities e.g. by way of buttons on the device, cable input or contactless communication. The signal receiver unit may also include a transmitter for transmitting sensor signals or variables derived therefrom to a remotely located device.

In one embodiment, the sensor includes a sensor substrate having one or more channels arranged between adjacent elements of the sensor. Such a sensor is suitable for use with an absorbent article having super absorbent material arranged correspondingly with a channel, in the article, so as to draw fluid from the one or more channels in the sensor substrate. Preferably, the sensor is provided on a flexible substrate affixable, by adhesive or other means, to an absorbent article wearable by a user.

The sensor elements detect wetness at various identifiable locations with respect to the absorbent article including toward the front of the absorbent article, toward the rear of the absorbent article, toward a side of the absorbent article, and substantially centrally of the absorbent article. Desirably, the pattern of sensor elements facilitates improved detection of moisture from a user in a range of positions including standing, sitting, lying prone, lying supine and lying on the side.

In one embodiment, the processor is configured to classify a possible form of incontinence suffered by a patient monitored by the system, such as urinary, fecal, dribble, stress, overflow, urge, mixed urinary (MUI), total and functional incontinence. The processor may also recognise and/or predict lingering wetness in a region of an absorbent article.

The processor may be affixable to a sensor, an absorbent article or to a garment worn by a wearer of an absorbent article. Alternatively, the processor can incorporated into a central monitoring station adapted to receive sensor signals from a plurality of sensors associated with one or more absorbent articles. A pre-processor may also be associated with a sensor of an absorbent article, locally to the article.

Preferably, the processor is adapted execute an algorithm to reconfigure one or more mathematical models for use with one or more of a particular individual being monitored, a different sensor type and a different absorbent article type. This may be achieved by, for a training period using the particular individual, the different sensor type or the different absorbent article type, monitoring wetness at regular intervals by obtaining sensor signals and obtaining observation data, and reconfiguring the mathematical model so that there is satisfactory correlation between the estimates produced using the sensor signals and the reconfigured mathematical model, and the observation data obtained during the training period. Reconfiguring a mathematical model preferably involves employing an algorithm to determine one or more new parameters for the mathematical model e.g. using a linear regression technique.

Observation data includes measurements indicating an amount of wetness in the absorbent article and time of measurement. It may also include demographic information about the patient such as age and gender and patient information such as food and fluid intake and medication regimes.

According to another aspect of the present invention, there is provided a moisture monitoring system as described above, and further including a sensor applied to or incorporated into an absorbent article being monitored for use with one or more of a particular individual being monitored, a different sensor type and a different absorbent article type by, for a training period using the particular individual, the different sensor type and/or the different absorbent article type, monitoring wetness at regular intervals by obtaining sensor signals and obtaining observation data and reconfiguring the mathematical model so that there is satisfactory correlation between the estimates produced using the sensor signals and the reconfigured mathematical model, and the observation data obtained during the training period. Reconfiguring a mathematical model may involve determining new parameters for the mathematical model e.g. by application of a linear regression algorithm.

Brief description of the drawings

The present invention will now be described in greater detail with reference to the accompanying drawings. It is to be understood that the particularity of the accompanying drawings does not supersede the generality of the preceding description of the invention.

Figure 1 is a schematic diagram illustrating features of a moisture monitoring system according to an embodiment of the present invention.
Figure 2 is a flow diagram showing typical steps in using the monitoring system for continuous monitoring of patients for wetness, using a sensor.
Figure 3 is a flow diagram showing steps involving use of the invention for care planning.
Figure 4 is a flow diagram indicating the steps involved with calculating or re-calculating parameters of a mathematical model.
Figures 5a and 5b illustrate an example of a sensor used with an absorbent article, according to an embodiment of the present invention.
Figure 6 represents a sensor signal showing temperature versus time.
Figure 7 illustrates an embodiment of a sensor having a channel between adjacent sensor elements.
Figure 8 is a schematic illustration of a diaper or adult incontinence garment showing a pattern of sensor elements according to an embodiment of the invention.

provided on a flexible substrate affixable, by adhesive or other means, to an absorbent article wearable by a user.
The sensor elements detect wetness at various identifiable locations with respect to the absorbent article including toward the front of the absorbent article, toward the rear of the absorbent article, toward a side of the absorbent article, and substantially centrally of the absorbent article. Desirably, the pattern of sensor elements facilitates improved detection of moisture from a user in a range of positions including standing, sitting, lying prone, lying supine and lying on the side. Preferably, the sensor elements are also arranged to detect spread of moisture from a wetness event in two or more directions. The sensor may include sensor elements for detecting one or more of electrical conductivity, temperature, pressure, pH, odour, gas and presence of a biological or chemical marker in exudate and location of exudate.
According to another aspect of the present invention, there is provided a method for monitoring moisture in an absorbent article including the steps of receiving one or more sensor signals associated with the absorbent article, the sensor signals indicating wetness in the absorbent article, using the processor to analyse the sensor signals using an algorithm, and characterising a wetness event in the absorbent article, characterised in that the algorithm applies one or more sensor signals to a pre-determined mathematical model to characterise a wetness event in an absorbent article and determine a range of predictions based on patterns identified from the sensor signals, and characterised in that parameters of the mathematical model are determined over a training period by the processor (i) continually receiving sensor signals indicating wetness and upon every variation in sensor values, receiving observation data corresponding to (a) changing the pad, (b) examining the pad, and (c) weighing the pad; (ii) performing a regression analysis to formulate parameters for the mathematical model; (iii) feeding the parameters back into the mathematical model to determine a confidence level indicating how accurately the mathematical model estimates the actual events defined by the observation data (406); and (iv) repeating steps (i) to (iii) until an acceptable confidence level is reached. A method for devising a mathematical model is also disclosed.
Characterising a wetness event preferably involves ascertaining one or more of an estimated volume of exudate in a wetness event and the nature of the exudate although it may also involve determining whether predefined criteria, defined by a mathematical model, have been met. A user may be notified automatically if one or more predetermined notification criteria are met.
Preferably, the algorithm executing the predetermined mathematical model receives as inputs one or more variables derived from the one or more sensor signals and these variables may be derived automatically using a processor as described above. The method may also include maintaining a toileting or voiding diary, being a log of monitored wetness events, also referred to as a bladder chart. A toileting or voiding schedule may also be derived for an individual being monitored, based on wetness events monitored using the monitoring system.

The method may also include predicting, based on a derived toileting or voiding schedule, when an individual is likely experience a wetness event which meets pre-defined criteria for manual checking and this can streamline patient care. The method also facilitates reconfiguring of one or more mathematical models for use with one or more of a particular individual being monitored, a different sensor type and a different absorbent article type by, for a training period using the particular individual, the different sensor type and/or the different absorbent article type, monitoring wetness at regular intervals by obtaining sensor signals and obtaining observation data and reconfiguring the mathematical model so that there is satisfactory correlation between the estimates produced using the sensor signals and the reconfigured mathematical model, and the observation data obtained during the training period. Reconfiguring a mathematical model may involve determining new parameters for the mathematical model e.g. by application of a linear regression algorithm.

The processor may be configured to receive data (either entered manually or automatically by, for example, scanning a barcode on a diaper) pertaining to known features of a diaper or incontinence garment being worn by a patient. The features may include the volume, type or brand of the diaper/garment, and the location of the sensors embedded therein. This data enables the processor to identify the type of pad and devise or apply a suitable mathematical model which when used in combination with the data received from the sensor(s) can enable the processor to perform powerful analysis. Because the processor uses wetness and e.g. location data sampled over successive periods; and algorithms using mathematical models to characterise wetness events, it is also able to characterise phantom events or noise, which may result from the patient moving or from intermittent brief interference from other components in the system, and disregard these artefact points.

To characterise the volume of an event, the algorithm applies one or more variables derived from the sensor signals of an individual's absorbent article to a mathematical model which estimates the volume of liquid in the event. The variables derived from the sensor signals may include one or more of: area under a sensor signal curve (e.g. signal magnitude versus time); highest sensor signal value in a predetermined time period; maximum value of a leading edge of the sensor signal; the rate of decay of sensor signal after a leading edge; volume estimated in a previous event; time of onset; time of termination of an event; duration; time of day; and time elapsed since the last detected wetness event; although it is to be understood that this list is not exhaustive.

In addition to volume (or instead of), the algorithm may be adapted to characterise other aspects of wetness events such as the nature of the exudate (i.e. urinary or fecal) and whether a series of wetness events can be classified into a particular type of incontinence such as stress, urge, fecal, overflow, mixed urinary (MUI), dribble, functional and total incontinence. This can be achieved by applying a suitable mathematical model developed by the same means as the models used to characterise different voiding volumes.

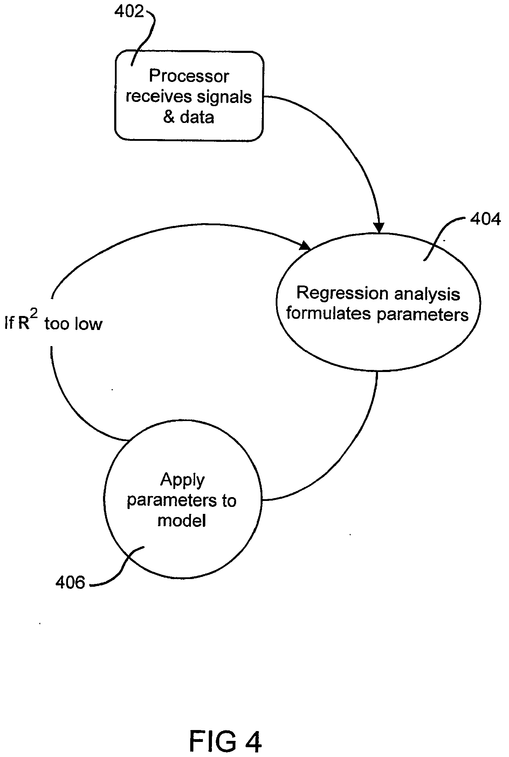

Referring now to Figure 4, there is shown a flow diagram indicating steps involved in an algorithm calculating and/or recalculating parameters of a mathematical model to characterise wetness events with maximum accuracy and/or to optimise its performance.

For a training period, e.g. 3 days, a patient is monitored for wetness. This may involve continually monitoring sensor signals for indications of wetness and upon every variation in sensor values, obtaining observation data by changing the pad, examining the pad and weighing the pad. Additional observation data may be collected such as amount and time of fluid and food intake, as these variables influence the patient's continence function and are therefore potentially influential variables in the mathematical model.

In a step 402, the collected sensor signals and observation data are received by the processor. In a step 404, the processor executes an algorithm performing a regression analysis to formulate parameters for the mathematical model. In a step 406, these parameters are fed back into the mathematical model and a confidence level is determined which indicates how accurately the mathematical model estimates the actual events defined by the observation data. If the confidence level is acceptable (e.g. above $R^2$ - 0.6) then the parameters are accepted and the model updated. If the confidence level is too low, a further regression analysis is performed and the confidence level checked again. The algorithm repeats the regression analysis process until an acceptable confidence level is reached.

The same method may be applied to re-calculate parameters of the model. Calculating and recalculating the parameters of the mathematical model utilised by the system is useful for a number of reasons. Firstly, it enables the establishment of an initial mathematical model for predicting particular types of events. Secondly, it allows the system to continually improve the accuracy with which it predicts a patient's continence function and therefore, the efficiency with which care practices can be implemented. Thirdly, by reconfiguring the mathematical model, the system can be adapted to work with different absorbent pads having different absorbent characteristics. In this way, the algorithm can "learn" the characteristics of the pad.

Similarly, the system can adapt to use with additional and/or different sensor types. Again, the ability of the system to "learn" the behaviour of different sensors and sensor elements makes the system adaptable to new products and technologies which will improve accuracy and sensitivity, without the need for a major overhaul of the software employed by the processor. Alternatively/additionally, the processor may re-define one or more mathematical models to suit new sensors, sensor elements or absorbent articles. The need to re-define a mathematical model can be minimised by use of relatively generic code, although this can result in slower calculations.

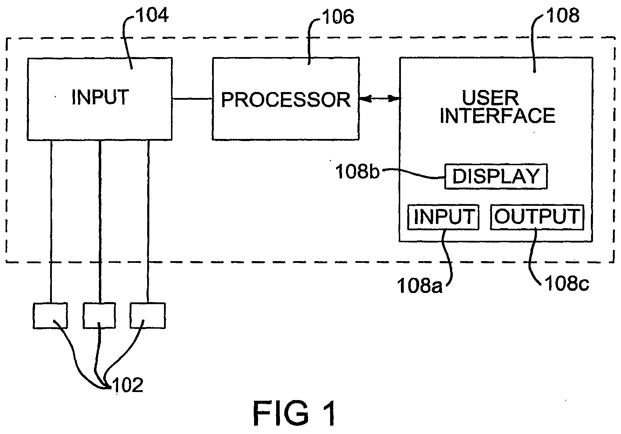

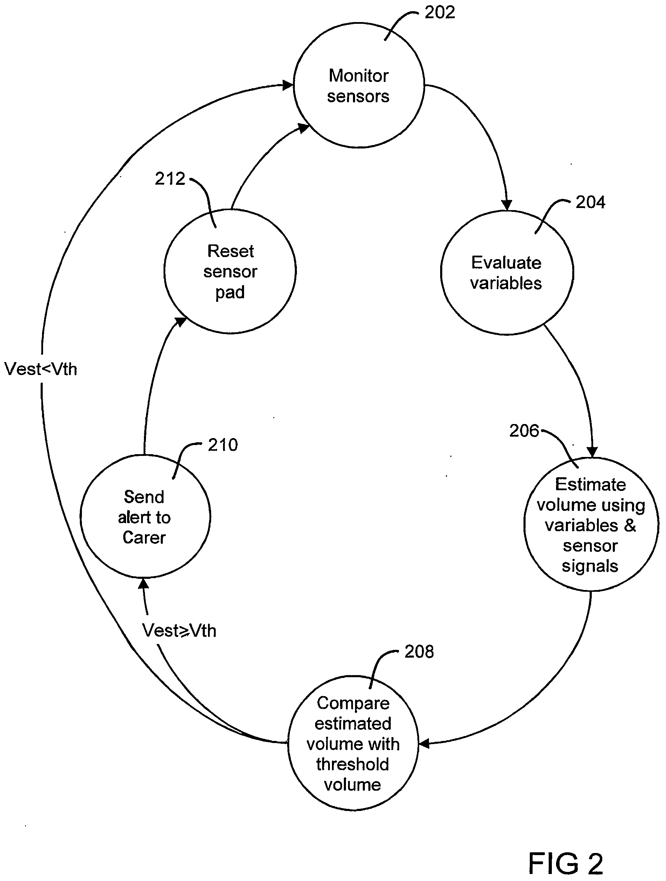

The moisture monitoring system of the present invention can be used to monitor incontinence sufferers more efficiently than existing systems. Figure 2 is a flow diagram illustrating typical steps involved in using the monitoring system for continuous monitoring of patients for wetness using sensors. In a step 202, the system monitors sensors applied to absorbent articles worn by patients in a care institution. If a sensor signal value exceeds an initial trigger value, in a step 204 the processor 106 (Fig 1) derives variables from received sensor signals which, in a step 206 are used as inputs to an algorithm using a pre-determined mathematical model to estimate a volume of exudate in a wetness event. The mathematical model may be determined using any suitable statistical modelling technique such as regression analysis. In this example, the algorithm applies a mathematical model to estimate volume of exudate using the equation:

$$Volume - 0.3x(\mathrm{Pr}\,ofile\_Area) + 2.4\,x(Patient\_Weight) - 0.6x(Patient\_Age)$$

$$(Eq\ 1)$$

Profile_Area is the area under a curve of sensor signal versus time for a sensor element monitoring exudate conductivity. Eq 1 gives a confidence factor ($R^2$) of 0.63. It is to be understood, however, that Eq 1 is just one example of a mathematical model which may be used to characterise wetness events and that other models may be derived with also exhibit a satisfactory level of confidence.

In a step 208 the processor executes an algorithm to compare the estimated volume with a pre-defined threshold level. If the estimated volume is less than the threshold, the processor continues to monitor the sensor signals. If the estimated volume exceeds the threshold amount, then in a step 210 the processor sends an alert to a carer. Once a carer is alerted, the carer attends to the resident and may choose to change the absorbent article and in a step 212, the processor detects that the sensor has been disconnected from the system and resets the sensor data.

The threshold volume used by the processor to alert a carer may be a "qualifying amount" e.g. indicated as small, medium or large or a quantifying amount being a pre-defined volume e.g. 50ml.

Preferably, the processor may also execute an algorithm to compare the estimated volume with a known estimated capacity of the diaper to give carers an indication of when the diaper is likely to become saturated with exudate so that it can be changed before a saturating wetness event occurs and the patient is made to feel uncomfortable by excess wetness.

The processor may also monitor the total amount of accumulated moisture in a series of wetness events in a single absorbent article and provide an indication to a carer as to when the absorbent capacity of the garment has been or is likely to be reached, to prompt the carer to change the garment for the patient's comfort and wellbeing.

Users may enter data, including patient specific demographic data such as gender, age, height and weight via user interface 108. As indicated in Equation 1 above, these data can also be utilised by the algorithm to estimate e.g. volume. Other entered data may include medical data, i.e. medication, amount of fluid and food intake, details of known conditions, recent surgeries, years in assisted care, years wearing an incontinence garment, continence function if known, and mental condition.

The processor 106 may be incorporated into a central monitoring station such as a nurse's station. The processor may also integrate with or be incorporated into existing nurse call and remote patient monitoring systems controlled at the nurse's station. The processor may also be integrated with other care management systems for streamlining access to non-sensor related data contained within other care management systems such as, for example, fluid and food intake, patient relocation, showering, toileting, surgeries etc.

User interface 108 may also include a transmitter which sends alerts to communication devices such as pagers or nurse phones carried by carers to indicate that there has been a wetness event, or that one is due to occur, or that physical inspection of the patient is required or due. In addition to the detection of wetness events which are estimated to exceed a threshold amount, these conditions warranting physical inspection may include when exudate is fecal in nature or when sensors detect blood, a parasite or a biological or chemical marker in the urine or faeces.

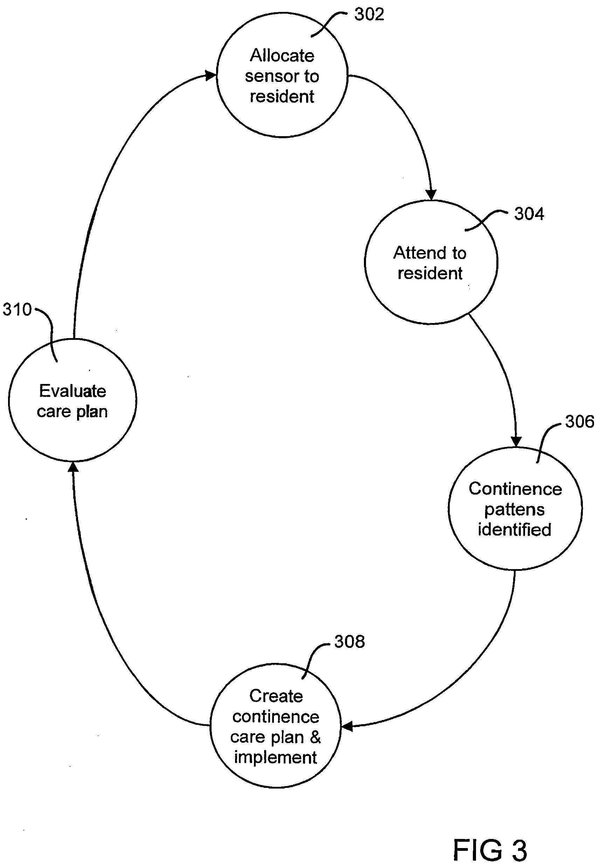

Figure 3 illustrates another use of the invention, where the moisture monitoring system is used in care planning to evaluate and plan the regularity and timing of a carer's manual checking of an individual's continence, and to schedule toileting. The care plan is based on an assessment performed using the monitoring system.

In a step 302, a sensor is allocated to a patient. The sensor has a transmitter unit attached and in a step 304 the patient is monitored for wetness for a continuous period of, for example, 3 to 5 days. During that period, the patient participates in usual activities and the patient is physically checked for wetness by a carer regularly, e.g. every hour. When a sensor signal received by the processor indicates a presence of exudate, an alert is sent to a carer who attends to the patient, changing the pad. Each time the carer checks or attends to the patient, observation data is recorded which includes the nature and amount of exudate (e.g. volume or mass obtained by weighing a soiled pad) and the time of observation.

In a step 306, observation data is used, along with a log of the sensor signals received at the input, to identify patterns in the patent's continence activity. In a step 308 the processor derives automatically, using an algorithm employing another mathematical model, a continence care plan based on the pattern, i.e. frequency and repetition of monitored events. The care plan includes a voiding or toileting schedule which statistically predicts wetness events based on the observed pattern. This is used by carers to plan the regularity (e.g. times of day) that a patient is to be manually checked for wetness and/or assisted with toileting and to plan when to empty the bladder or bowel, prior to periods in which a patient is known to have a pattern of incontinence events. Normal care of the patient can then take place without the need to continually monitor using a sensor.

The voiding schedule anticipates when a wetness event is statistically likely to occur and this can be used to automatically generate an audible and/or visible alert for a carer (e.g. presented on a screen of the user interface 108 or transmitted to a pager or the like) to attend to the patient by assisting with manual toileting or to change the patient's incontinence garment.

It is recommended that the toileting/voiding schedule is re-evaluated periodically (step 310) to maintain its accuracy, in keeping with changes in the patient's continence patterns. Re-evaluation may take place for example every 3, 6 or 12 months, or whenever actual wetness events do not correspond well with those anticipated by the voiding schedule.

In another use of the invention, the moisture monitoring system includes a log for recording wetness events detected by sensors including the volume, time and nature (urinary and/or fecal) of each event. These data are used to produce a bladder diary. These data may also be combined with details entered e.g. at the user interface 108 which relate to food and fluid intake (amount, kind and time), toileting and also any particular activities that the patient has undertaken.

The log may manifest in a memory device in communication or integrated with the processor. The processor may be located centrally and receive sensor signals relating indicative of wetness of a number of absorbent articles worn by different patients. Alternatively, there may be a pre-processor executing the algorithm located near the sensor, on the absorbent article. That is, the sensor and the part of the processor performing the analysis may be a provided together with the sensor. In such arrangement, the pre-processor may also incorporate a transmitter for transmitting data from the pre-processor to e.g. a central monitoring system which may include a display.

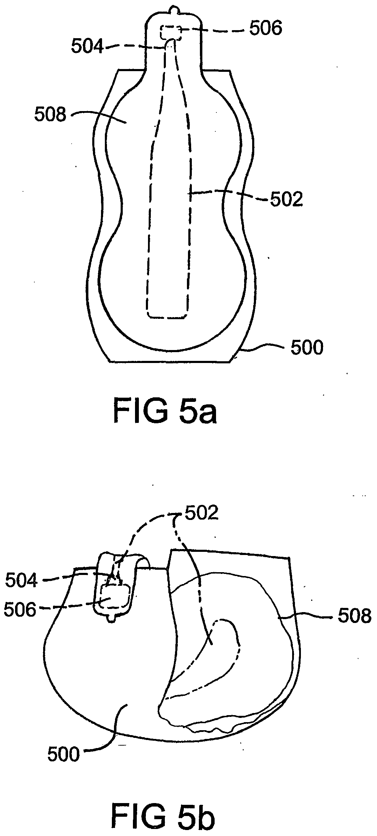

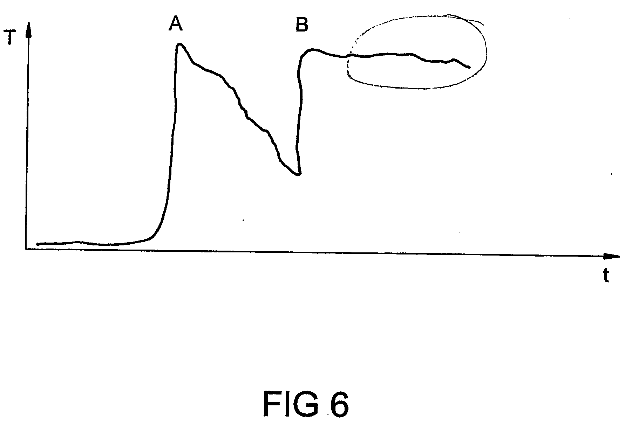

Referring now to Figures 5a and 5b, there is shown a schematic diagram of a sensor 502 according to an embodiment of the invention, applied to an absorbent article 500. The sensor 502 has a sensor element (shown in broken lines) which exhibits a change in conductivity when moisture is present, although other variables such as temperature could be used to detect moisture, as indicated in Figure 6.

Figure 6 is a graph of temperature versus time. The rise in temperature at point A is indicative of a wetness event and the rate of decline indicates that the temperate moisture is being drawn away from the sensor element, into the absorbent article. The second leading edge peaking at point B indicates the occurrence of a second wetness event and the signal is sustained. This is typically indicative of a situation in which the absorbent article must be changed e.g. because of the size of the wetness event, because the article has reached its absorbent capacity, or a fecal event has occurred in which the exudate cannot be drawn into the absorbent layers of the article.

Returning to Figures 5a and 5b, an embodiment of the invention is shown in which the sensor elements 502 extend beyond a front edge of the absorbent article 500. In this arrangement, a connector 504 is also provided to which a signal receiver unit 506 may be attached. The signal receiver unit may consist of a storage component which records the time and magnitude of sensor signals. Alternatively/additionally, it may include a transmitter which conveys the time and magnitude of sensor signals to a remotely located processor. Alternatively/additionally, the signal receiver unit may include pre-processing means for executing an algorithm performing analysis of the sensor signals which are then stored locally for downloading and/or transmitted to a remote processor which conveys alerts to carers, formulates bladder diaries, voiding schedules or the like. In such arrangement, the signal receiver unit (i.e. pre-processor) may also include means for receiving data relating to a patient's toileting activities. The data may be received wirelessly via a contactless communication device, by a cable connection to an input device or other suitable means for example buttons or the like on the signal receiver unit itself, worn by the patient.

Preferably, signal receiver units 506 are re-useable, and are releasably connectable to the sensors via connectors 504. This connection may utilise any suitable connection means such as a male-female dual-in-line (DIL) connector or the like, as would be known to a person skilled in the relevant art. The signal receiver units may be attached to an absorbent article or to clothing worn by a patient in a manner which is comfortable for the patient to wear, and is also sufficiently robust to minimise the risk of damage or removal while in use. When the diaper/incontinence garment is changed, the signal receiver unit may be disconnected from the soiled sensor, cleaned and attached to a sensor on a new diaper/incontinence garment.

Alternatively, the signal receiver units and sensors may be disposable and incorporated into a diaper or absorbent article during manufacture. In this arrangement, the signal receiver unit may not be visible so the sensor may be activated by a switch or button which is felt through the layers of the diaper. Alternatively, a radio-frequency or other contactless system may be used to activate the device and/or transmit sensor signals to a central monitoring station. In a further alternative embodiment, all parts of the monitoring system are re-useable, although this may create hygiene problems and be undesirable for individuals left with the task of cleaning the components.

In the embodiments illustrated in Figures 5a and 5b, a cover layer 508 is provided over the sensor elements. In regions of the cover layer affected by exudate, it is preferred that the cover layer material is liquid permeable so that any moisture resulting from a wetness event can be drawn into the absorbent layers of garment 500. In a preferred embodiment, a flap or pouch is provided to contain the signal receiver unit (which may also provide a transmitting/pre-processing/memory functions). The flap or pouch may be provided by a portion 508a of the cover layer which extends beyond and folds over the front edge of the absorbent article and can be fastened in place by adhesive, Velcro® or other means. The flap or pouch deters individuals, particularly those with forms of dementia or mental illness from tampering with the unit. Preferably, the sensor and absorbent article are arranged in such a way that the signal receiver unit is attachable thereto outside the absorbent article.

The sensor and other components which are located on the diaper (e.g. transmitter, pre-processor) may be powered by a small battery or electronic component storing energy. Alternatively, the sensor may include or be part of an RFID or other passive device. To conserve power, the transmitter/pre-processor may deactivate when there has been no wetness event for a predetermined length of time. The devices may be reactivated when a wetness event occurs.

The processor analyses signals received from the sensors to characterise wetness events which are detected for each patient. Characterisation of wetness events by the processor may include characterising the cause of a wetness event by making a distinction between wetness resulting from incontinence, perspiration or other leakage or discharge which may occur due to bedsores or decubtious ulcers which can develop in immobile patients.

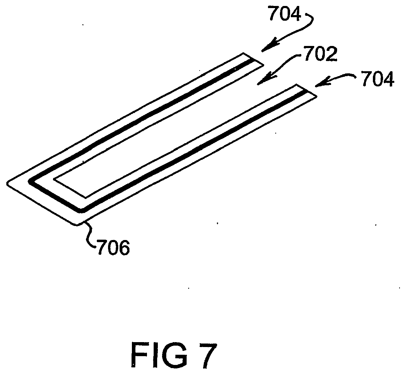

A sensor 102 may be incorporated into a pad, diaper or adult incontinence garment when manufactured, or it may be provided separately and attached to an "off the shelf" diaper by way of adhesive or other fixation means. For the latter, sensor elements are provided on a substrate which may be liquid permeable so that exudate released by the wearer passes through the substrate (activating the sensor elements) and is drawn away from the user into the absorbent layers of the diaper. One or more pores and/or channels may be provided in the sensor substrate to facilitate drawing of exudate away from the skin surface into the diaper. Figure 7 illustrates one such embodiment, where there is an elongate channel 702 provided between two elongate sensing elements 704 on a substrate 706 of a sensor. It may be desirable to use a sensor having a substrate of this kind with an absorbent article with super absorbent material correspondingly arranged in the article so as to draw fluid from the one or more channels in the sensor substrate into the absorbent layers and away from the wearer.

In one embodiment, sensors 102 are conductive elements. When an electrolyte such as urine contacts the conductive elements in sufficient quantity, a conductive bridge is formed between the elements and this can be detected by monitoring one or more electrical characteristics of the elements such as resistance or conductance, capacitance or the like. The conductive elements may be formed using any suitable conductive materials or combinations of materials including gold, copper, silver, conductive inks, polymers, tapes, resins and threads, other suitable conductive polymeric materials, conductive film, fibres or electrodes including, for example, an inert metal. Alternatively/ additionally, the sensor elements may detect changes in temperature, pH odour, gas, or the presence of blood or a chemical or biological marker in exudate to indicate a presence of moisture.

Production of the sensor may utilise a range of manufacturing methodologies. One example is screen printing or etching which can be employed to deposit the sensing elements on a suitable substrate. In one form, the sensor may be provided in the form of a flexible printed circuit board formed on a Mylar or other suitable flexible substrate. For three-dimensional arrays, the sensor elements may be deposited on a number of substrate layers which are then bonded into a multilayer liner. For sensor elements incorporated into diapers, depositing the layers on the various absorbent layers of the diaper can be integrated into the diaper manufacturing process.

The sensor elements may be elongate or provided in the form of grids, dots or the like, arranged in a pattern along and/or in the diaper or a pad or liner attachable thereto. By utilising, for example, screen printing or etching techniques, effective patterns can be designed and printed in layers of the sensor quickly and accurately. Advantageously, screen printing can deposit conductive polymers, inks and the like in very fine lines or grids between which exudate including urine and faeces may be absorbed into deeper, more absorbent layers. This enables conductive elements of a sensor to be incorporated into a diaper or absorbent article without significantly affecting the performance of the diaper.

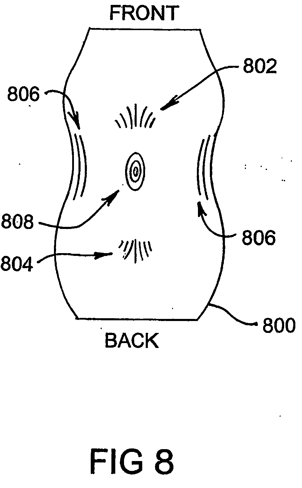

The sensor elements are preferably provided in a quantity and pattern sufficient to enable detection of moisture in different locations in an absorbent article being worn by a user. The pattern may be a two-dimensional pattern in which sensor elements are provided in a single layer or in a three-dimensional pattern. The pattern of sensor elements is preferably such that the elements are focussed in regions of the article where there is a greater likelihood of them being affected by a wetness event. Figure 8 is a schematic drawing of a diaper 800 laid flat, showing one example of a pattern of sensor elements which may be suitable. Each of the sensor elements may be uniquely identified enabling sensor signals to convey to the processor data indicating that wetness is present, as well as the location of the sensor element(s) detecting the wetness. This enables the processor to determine where in the absorbent article and the extent to which the wetness has occurred. Spread of wetness may also be identified.

In one embodiment, the sensor has a plurality of layers and the sensor elements are arranged in a three-dimensional pattern within the layers. A three-dimensional array is advantageous for a number of reasons. Firstly, absorbent articles such as diapers are flexible in nature and therefore prone to folding or scrunching particularly in regions around the legs. To circumvent a problem in which 2 or more conductive elements of a sensor are caused to "short" together as a result of a fold in the article or movement of a wearer, adjacent conductive elements may be placed in alternate layers of the sensor, separated by an electrically insulating permeable layer to prevent shorting in the absence of wetness.

Secondly, by positioning sensor elements in different layers, it is possible for the sensor to convey additional location data to the processor relating to the depth at which moisture is detected. This is particularly important for sophisticated diapers and incontinence garments which are multi-layered in their construction and designed with super absorbent and "wicking" properties to draw wetness away from the wearer and direct it to chambers or zones in the absorbent layers where it is retained. Positioning sensor elements in or near various absorbent layers of the article can convey further relevant data to the processor which may relate to, for example, the degree of wetness (or fullness) of a storage chamber within a diaper. Also, elements located at various depths allow the system to monitor the absorption of fluid into a diaper. Thus, the sensor will not require 'pooling' of moisture to detect wetness. This is especially useful in view of the fact that most modern absorbent garments are manufactured to maximise the absorption of liquid away from the skin.

As indicated above, the sensor elements are arranged in a pattern which maximises the ability to detect relevant data, for use in characterising wetness events. For example, as illustrated in Figure 8, the pattern may provide sensor elements more densely in a region toward the front of the absorbent article (802), to the rear of the absorbent article (804), and around the leg openings (806) and in the centre, between the leg opening (808), where liquid is likely to drain. Positioning the sensor elements in this way improves the detection of urinary wetness which normally occurs toward the front of an absorbent article, detection of faecal wetness which normally occurs to the rear of an article, and detection of wetness resulting from perspiration which can frequently occur, for example, toward the sides in the crotch area near the crease of the wearer's legs, and toward the middle of the diaper.

The sensor may also provide means to detect temperature, pressure, presence of a gas or odour in the absorbent article and/or the presence of a biological or chemical marker indicating presence of bacteria, sugar, parasites or the like in the urine or faeces. This is particularly useful for patients who lack the ability to control where and/or when a voiding event will occur. Data pertaining to these further parameters can also be used, in combination with signals from the conductive elements to further characterise a wetness event, provide a diagnostic indicator, or at least give a carer an early indication that a particular patient is in need of further attention. Other sensor elements may also be incorporated to indicate whether the patient is moving or in a sitting. Lying or standing position.

The sensor signals may be logged regularly, say, every 100 milliseconds or sufficiently frequently to reliably and accurately detect and distinguish an event. Signals received by the processor can reveal data indicating for example (i) detection of wetness and (i) location of the detected wetness. These signals can vary over time, as liquid is absorbed though the diaper and further wetness events occur. By monitoring these signals in time, it is possible for the processor to derive further useful parameters such as volume of exudate in an event and total volume absorbed, using mathematical modelling.

Also, the volume of exudate released can be computed using such factors as the distance between sensor elements detecting the wetness, the rate of transfer of moisture between these elements and the absorption properties of the materials used. These materials may include polymer fibres, natural fibres, gels, textiles, fabrics, papers or a combination of these materials.

The processor may also be programmed with or can interrogate a database of "event signatures" or models characterising certain wetness events and correlate the sensors signals with the event signatures/models to characterise wetness events which are detected. The models may be embodied in any form including mathematical models as described above, graphs or look up tables.

Advantageously, by including laterally placed sensor elements in the sensor pattern, incontinence events can be detected irrespective of whether the patient is in the sitting, lying or standing position. For instance, if the patient is lying on his side, laterally located sensor elements are more likely to detect urinary exudate than the frontal elements which would be activated if the patient was standing or sitting.

Lingering wetness may be indicated by failure of the signal to recover to a normal level. A prolonged high sensor signal value may indicate the presence of faecal matter which, unlike urine, may not be drawn into the absorbent layers of the diaper but remains in contact with the sensor elements. Detection of a faecal event should be accompanied by an alert to a carer to change the diaper so as to avoid prolonged wetness and discomfort. A lingering wetness may also be indicative of a full diaper, resulting from inability of the diaper to draw any more urine away from the wearer. This condition should also be communicated to a carer.

It is to be understood that various modifications, additions and/or alterations may be made to the parts previously described without departing from the ambit of the present invention as defined in the claims appended hereto.

**Claims**

1. A moisture monitoring system for monitoring wetness in one or more absorbent articles, the system including:

   an input (104) for receiving one or more sensor signals indicative of a presence of wetness in an absorbent article;
   a processor (106) for processing the one or more sensor signals; and
   a user interface (108) for communicating with a user of the system;
   wherein the processor (106) is adapted to execute an algorithm for performing an analysis of the signals, whereby the algorithm applies the received sensor signals to a pre-determined mathematical model to characterise a wetness event in an absorbent article and to determine a range of predictions based on patterns identified from the sensor signals, and
   **characterised in that** parameters of the mathematical model are determined over a training period by the processor (i) continually receiving sensor signals indicating wetness and upon every variation in sensor values, receiving observation data corresponding to (a) changing the pad, (b) examining the pad, and (c) weighing the pad; (ii) performing a regression analysis to formulate parameters for the mathematical model; (iii) feeding the parameters back into the mathematical model to determine a confidence level indicating how accurately the mathematical model estimates the actual events defined by the observation data; and (iv) repeating steps (i) to (iii) until an acceptable confidence level is reached.

2. A moisture monitoring system according to claim 1 wherein the processor is adapted to execute an algorithm to devise automatically a mathematical model for characterising a wetness event in an absorbent article and optionally, to reconfigure one or more mathematical models for use with one or more of a particular individual being monitored, a different sensor type, and a different absorbent article type, by employing an algorithm to determine one or more parameters for the mathematical model and obtaining sensor signals and observation data over a training period using the particular individual being monitored, a different sensor type, or a different absorbent article type, and reconfiguring the mathematical model so there is satisfactory correlation between a) estimates produced using sensor signals and the mathematical model; and b) the observation data obtained during the training period.

3. A moisture monitoring system according to claim 1 or claim 2, wherein the algorithm applies variables derived from the one or more sensor signals to the mathematical model for the purpose of characterising a wetness event.

4. A moisture monitoring system according to claim 1 wherein characterising a wetness event in an absorbent article includes determining one or more of:

   (i) an estimated volume of exudate in a wetness event; and
   (ii) the nature of exudate in a wetness event.

5. A moisture monitoring system according to claim 1 wherein the algorithm applies variables derived from the one or more sensor signals to a pre-determined mathematical model to characterise a wetness event.

6. A moisture monitoring system according to claim 5 wherein variables derived from the sensor signals are selected from the group including:

   (i) area under a sensor signal curve;
   (ii) highest sensor signal value in a predetermined time period;
   (iii) maximum value of a leading edge of the sensor signal;
   (iv) rate of decay of sensor signal after a leading edge;
   (v) a volume estimated in a previous wetness event;
   (vi) time of onset of a wetness event;
   (vii) time of termination of a wetness event; and
   (viii) duration of a wetness event;
   (ix) time of day of a wetness event; and
   (x) time elapsed since last wetness event.

7. A moisture monitoring system according to any one of the preceding claims wherein the processor is configured to determine a wetness event prediction including one or more of:

   (a) a likelihood of an imminent wetness event;

(b) an estimate of when a wetness event is likely to occur;

(c) an estimate of a degree of fullness of an absorbent article; and

(d) an estimate of when an absorbent article is likely to reach its absorbent capacity.

8. A moisture monitoring system according to any one of the preceding claims wherein the processor is configured to provide one or more of:

(a) a toileting or voiding diary;

(b) a toileting or voiding schedule for an individual, based on wetness events monitored using the monitoring system e.g. over a number of days;

(c) an indication of when an individual is likely to experience a wetness event which meets pre-defined criteria for manual checking based on a derived toileting or voiding schedule;

(d) a communication alert generated and transmitted automatically to a carer when one or more pre-defined criteria for manual checking are satisfied;

(e) a classification indicating a possible form of incontinence suffered by a patient monitored by the system, the form of incontinence being selected from the group including urinary, fecal, dribble, stress, overflow, urge, mixed urinary (MUI), total and functional incontinence; and

(f) an indication of detected or predicted lingering wetness in a region of an absorbent article.

9. A moisture monitoring system according to any one of the preceding claims wherein the processor is adapted to receive patient data for use with the mathematical model, the patient data including one or more data selected from a group including but not limited to weight, height, age, gender, pre-existing conditions, medications and recent surgeries.

10. A moisture monitoring system according to any one of the preceding claims adapted to reconfigure one or more mathematical models for use with one or more of a particular individual being monitored, a different sensor type and a particular absorbent article type, by:

for a training period using the particular individual, the different sensor type or the particular absorbent article type, monitoring wetness at regular intervals by obtaining sensor signals and obtaining observation data; and reconfiguring the mathematical model so that there is satisfactory correlation between the estimates produced using the sensor signals and the reconfigured mathematical model, and the observation data obtained during the training period.

11. A moisture monitoring system according to claim 10 wherein reconfiguring a mathematical model involves one or more of:

(a) determining one or more new parameters or coefficients for the mathematical model; and

(b) application of a linear regression algorithm.

12. A moisture monitoring system according to claim 10 or claim 11 wherein the observation data includes one or more of:

(a) demographic information; and

(b) patient information.

13. A moisture monitoring system according to any one of the preceding claims, further including a sensor (502) applied to or incorporated into an absorbent article (500) being monitored for wetness using the moisture monitoring system, the sensor including a plurality of sensor elements (502) arranged in a pattern which provides an improved ability to detect wetness, preferably with more sensor elements in regions having higher propensity for variable moisture or temperature.

14. A moisture monitoring system according to claim 13, wherein the one or more sensor elements extend beyond an edge thereof and further including a cover layer (508) over the sensor elements, the cover layer extending beyond an edge of the absorbent article (500) and including means for enclosing a signal receiver unit (506) attachable to one or more of the sensor elements.

15. A moisture monitoring system according to claim 13 or claim 14 further including a signal receiver unit (506) connectable to the sensor elements (502), wherein the signal receiver unit includes storage means for storing sensor signals collected over a period of time and preferably wherein the signal receiver unit includes means for receiving data relating to a patient's toileting activities.

16. A moisture monitoring system according to any one of claims 13 to 15 wherein the pattern includes sensor elements (502) positioned toward the sides of the absorbent article (500), near an opening for receiving a leg of the wearer.

17. A moisture monitoring system according to any one of claims 13 to 14 wherein the sensor (502) includes a sensor substrate, the sensor substrate having one or more channels arranged between adjacent elements of the sensor so that in use with an absorbent article (500) having super absorbent material arranged therein, fluid is drawn from the one or more channels in the sensor substrate.

18. A moisture monitoring system according to any one of claims 13 to 17 wherein the sensor elements detect wetness at various identifiable locations with respect to the absorbent article, the locations selected from the group including:

   (a) toward the front of the absorbent article;
   (b) toward the rear of the absorbent article;
   (c) toward a side of the absorbent article; and
   (d) substantially centrally of the absorbent article.

19. A method for monitoring moisture in an absorbent article including the steps of:

   (a) receiving (402) at a processor one or more sensor signals associated with the absorbent article, the sensor signals indicating wetness in the absorbent article;
   (b) using the processor to analyse the sensor signals using an algorithm (404) ; and
   (c) characterising a wetness event in the absorbent article;
   **characterised in that** the algorithm applies one or more sensor signals to a pre-determined mathematical model to characterise a wetness event in an absorbent article and determine a range of predictions based on patterns identified from the sensor signals, and
   **characterised in that** parameters of the mathematical model are determined over a training period by the processor (i) continually receiving sensor signals indicating wetness and upon every variation in sensor values, receiving observation data corresponding to (a) changing the pad, (b) examining the pad, and (c) weighing the pad; (ii) performing a regression analysis to formulate parameters for the mathematical model; (iii) feeding the parameters back into the mathematical model to determine a confidence level indicating how accurately the mathematical model estimates the actual events defined by the observation data (406); and (iv) repeating steps (i) to (iii) until an acceptable confidence level is reached.

20. A method for monitoring moisture according to claim 19 wherein characterising a wetness event involves ascertaining one or both of:

   (i) an estimated volume of exudate in a wetness event; and
   (ii) the nature of the exudate.

21. A method for monitoring moisture according to claim 19 or claim 20 wherein the predetermined mathematical model has one or more variables derived from the one or more sensor signals.

22. A method of monitoring moisture according to any one of claims 19 to 21 further including one or more of:

   (a) deriving a toileting or voiding schedule for an individual, based on wetness events monitored using the system;
   (b) predicting, based on a derived toileting or voiding schedule, when an individual is likely experience a wetness event which meets pre-defined criteria for manual checking; and
   (c) reconfiguring the mathematical model for use with one or more of a particular individual being monitored, a different sensor type and a particular absorbent article type, by:

      (i) for a training period using the particular individual, the different sensor type and/or the particular absorbent article type, monitoring wetness at regular intervals by obtaining sensor signals and obtaining observation data; and
      (ii) reconfiguring the mathematical model so that there is satisfactory correlation between the estimates produced using the sensor signals and the reconfigured mathematical model, and the observation data obtained during the training period.

23. A method of monitoring moisture according to claim 22 wherein reconfiguring a mathematical model involves one or both of:

(a) determining new parameters for the mathematical model; and
(b) application of a linear regression algorithm.

24. A method of monitoring moisture according to claim 22 or claim 23 wherein the observation data further includes one or more of:

(a) demographic information; and
(b) patient information.

**Patentansprüche**

1. Ein Feuchtigkeitsüberwachungssystem für die Überwachung der Feuchtigkeit in einem oder mehreren absorbierenden Artikeln, das System schließt dabei Folgendes ein:

einen Eingang (104) für den Empfang eines oder mehrerer Sensorsignale, die das Vorhandensein von Feuchtigkeit in einem absorbierenden Artikel anzeigen;
einen Prozessor (106) für die Verarbeitung des einen oder der mehreren Sensorsignale; und
eine Benutzerschnittstelle (108) für die Kommunikation mit einem Benutzer des Systems;
wobei der Prozessor (106) so angepasst ist, dass er einen Algorithmus für die Durchführung einer Analyse der Signale ausführen kann, wobei der Algorithmus die empfangenen Sensorsignale auf ein vorbestimmtes mathematisches Modell anwendet, um ein Feuchtigkeitsereignis in einem absorbierenden Artikel zu charakterisieren und einen Vorhersagebereich, auf der Grundlage der aus den Sensorsignalen erkannten Muster, zu bestimmen, und
**gekennzeichnet dadurch, dass** Parameter des mathematischen Modells über eine Eingewöhnungszeit bestimmt werden, indem der Prozessor (i) kontinuierlich Sensorsignale empfängt, die Feuchtigkeit anzeigen, und bei jeder Änderung der Sensorwerte, Beobachtungsdaten empfängt, die (a) dem Wechsel des Polsters, (b) der Überprüfung des Polsters und (c) dem Wiegen des Polsters entsprechen, (ii) eine Regressionsanalyse ausführt, um die Parameter für das mathematische Modell zu entwickeln; (iii) die Parameter zurück in das mathematische Modell speist, um ein Vertrauensniveau zu bestimmen, das anzeigt, wie genau das mathematische Modell die tatsächlichen Ereignisse, die von den Beobachtungsdaten definiert sind, schätzt; und (iv) die Schritte (i) bis (iii) wiederholt, bis ein akzeptables Vertrauensniveau erreicht ist.

2. Ein Feuchtigkeitsüberwachungssystem gemäß Anspruch 1, wobei der Prozessor so angepasst, ist, dass er einen Algorithmus ausführt, um automatisch ein mathematisches Modell für die Charakterisierung eines Feuchtigkeitsereignisses in einem absorbierenden Artikel zu entwickeln und optional ein oder mehrere mathematische Modelle neu zu konfigurieren, die bei einem oder mehreren eines bestimmten zu überwachenden Individuums, einem unterschiedlichen Sensortyp und einem unterschiedlichen absorbierenden Artikeltyp eingesetzt werden sollen, durch Anwendung eines Algorithmus, um einen oder mehrere Parameter für das mathematische Modell zu bestimmen und Sensorsignale und Beobachtungsdaten über eine Eingewöhnungszeit zu erhalten, unter Verwendung des bestimmten zu beobachtenden Individuums, eines unterschiedlichen Sensortyps und eines unterschiedlichen absorbierenden Artikeltyps, und die Neukonfiguration des mathematischen Modells, damit eine zufriedenstellende Korrelation zwischen a) den durch die Verwendung der Sensorsignale und des mathematischen Modells entstandenen Schätzungen; und b) den Beobachtungsdaten, die während der Eingewöhnungszeit erlangt wurden, entsteht.

3. Ein Feuchtigkeitsüberwachungssystem gemäß Anspruch 1 oder Anspruch 2, wobei der Algorithmus Variablen anwendet, die von dem einen oder den mehreren Sensorsignalen in das mathematische Modell übernommen werden, um ein Feuchtigkeitsereignis zu charakterisieren.

4. Ein Feuchtigkeitsüberwachungssystem gemäß Anspruch 1, wobei die Charakterisierung eines Feuchtigkeitsereignisses in einem absorbierenden Artikel die Bestimmung eines oder mehrerer der folgenden Elemente einschließt:

(i) eine geschätzte Menge eines Exsudats in einem Feuchtigkeitsereignis und

(ii) die Art des Exsudats in einem Feuchtigkeitsereignis.

5. Ein Feuchtigkeitsüberwachungssystem gemäß Anspruch 1, wobei der Algorithmus Variablen anwendet, die von dem einen oder den mehreren Sensorsignalen in das vorbestimmte mathematische Modell übernommen werden, um ein Feuchtigkeitsereignis zu charakterisieren.

6. Ein Feuchtigkeitsüberwachungssystem gemäß Anspruch 5, wobei die von den Sensorsignalen abgeleiteten Variablen, ausgewählt sind aus der Gruppe, die Folgendes einschließt:

    (i) den Bereich unter der Sensorsignalkurve;
    (ii) den höchsten Sensorsignalwert in einer vorbestimmten Zeit;
    (iii) den Höchstwert einer Eintrittskante des Sensorsignals;
    (iv) die Verfallsgeschwindigkeit des Sensorsignals nach der Eintrittskante;
    (v) eine in einem vorherigen Feuchtigkeitsereignis geschätzte Menge;
    (vi) die Empfangsdauer eines Feuchtigkeitsereignisses;
    (vii) die Beendigungsdauer eines Feuchtigkeitsereignisses; und
    (viii) die Dauer eines Feuchtigkeitsereignisses;
    (ix) die Tageszeit eines Feuchtigkeitsereignisses; und
    (x) die seit dem letzten Feuchtigkeitsereignis vergangene Zeit.

7. Ein Feuchtigkeitsüberwachungssystem gemäß eines der vorhergehenden Ansprüche, wobei der Prozessor so gestaltet ist, dass er eine Feuchtigkeitsereignis-Vorhersage bestimmt, die eines oder mehrere der folgenden Elemente einschließt:

    (a) eine Wahrscheinlichkeit eines bevorstehenden Feuchtigkeitsereignisses;
    (b) eine Schätzung, wann ein Feuchtigkeitsereignis wahrscheinlich auftreten wird;
    (c) eine Schätzung eines Sattheitsgrads eines absorbierenden Artikels; und
    (d) eine Schätzung, wann ein absorbierender Artikel wahrscheinlich seine Absorptionskapazität erreicht hat.

8. Ein Feuchtigkeitsüberwachungssystem gemäß eines der vorhergehenden Ansprüche, wobei der Prozessor so gestaltet ist, dass er eines oder mehrere der folgenden Elemente bereitstellt:

    (a) ein Toilettengang- oder Harnabgangs-Tagebuch;
    (b) einen Toilettengang- oder Harnabgangsplan für ein Individuum, auf der Grundlage eines Feuchtigkeitsereignisses, das unter Verwendung des Überwachungssystems, z.B. über eine bestimmte Anzahl an Tagen, überwacht wird;
    (c) eine Anzeige, wann ein Individuum wahrscheinlich ein Feuchtigkeitsereignis erfährt, das die vorbestimmten Kriterien für die manuelle Prüfung, auf der Grundlage eines abgeleiteten Toilettengang- oder Harnabgangsplans, erfüllt;
    (d) ein Kommunikationsalarm, der automatisch erzeugt und an einen Pfleger übermittelt wird, wenn ein oder mehrere vordefinierte Kriterien für eine manuelle Überprüfung erfüllt sind;
    (e) eine Klassifizierung, die eine mögliche Form einer Inkontinenz anzeigt, unter der ein vom System überwachter Patient leidet, die Form der Inkontinenz ist dabei ausgewählt aus der Gruppe, die Harn-, Stuhl-, Tröpfel-, Stress-, Überlauf-, Drang-, gemischte Harn- (MUI), Gesamt- und Funktionsinkontinenz einschließt; und
    (f) eine Anzeige einer erfassten oder vorhergesehenen fortbestehenden Feuchtigkeit in einem Bereich eines absorbierenden Artikels.

9. Ein Feuchtigkeitsüberwachungssystem gemäß eines der vorhergehenden Ansprüche, wobei der Prozessor so angepasst ist, dass er Patientendaten empfängt, um sie im mathematischen Modell zu verwenden, die Patientendaten schließen eine Datenangabe oder mehrere Datenangaben ein, ausgewählt aus der Gruppe, die Folgendes einschließt, aber nicht darauf beschränkt ist, Gewicht, Größe, Alter, Geschlecht, vorher vorhandene Zustände, Medikationen und kürzliche Operationen.

10. Ein Feuchtigkeitsüberwachungssystem gemäß eines der vorhergehenden Ansprüche, das so angepasst ist, dass es ein oder mehrere mathematische Modelle neu konfiguriert, um sie bei einem oder mehreren zu überwachen Individuen, einem unterschiedlichen Sensortyp und einem bestimmten absorbierenden Artikeltyp zu verwenden, durch:

die Verwendung des bestimmten Individuums, des unterschiedlichen Sensortyps oder des bestimmten absorbierenden Artikeltyps für eine Eingewöhnungszeit, die Überwachung der Feuchtigkeit in regelmäßigen Abständen durch Erfassen der Sensorsignale und der Beobachtungsdaten; und
die Neukonfiguration des mathematischen Modells, so dass eine ausreichende Korrelation zwischen den Schätzungen, die unter Verwendung der Sensorsignale und des neu konfigurierten mathematischen Modells erzeugt wurden, und den Beobachtungsdaten, die während der Einarbeitungszeit erfasst wurden, hergestellt wird.

11. Ein Feuchtigkeitsüberwachungssystem gemäß Anspruch 10, wobei die Neukonfiguration eines mathematischen Modells ein oder mehrere der folgenden Elemente einschließt:

   (a) die Bestimmung eines oder mehrerer neuer Parameter oder Koeffizienten für das mathematische Modell; und
   (b) die Anwendung eines linearen Regressionsalgorithmus.

12. Ein Feuchtigkeitsüberwachungssystem gemäß Anspruch 10 oder Anspruch 11, wobei die Beobachtungsdaten eines oder mehrere der folgenden Elemente einschließen:

   (a) demographische Informationen; und
   (b) Patienteninformationen.

13. Ein Feuchtigkeitsüberwachungssystem gemäß eines der vorhergehenden Ansprüche, das darüberhinaus einen Sensor (502) einschließt, der angewendet wird auf oder eingebunden ist in einen absorbierenden Artikel (500), der auf Feuchtigkeit überwacht wird, unter Verwendung des Feuchtigkeitsüberwachungssystems, der Sensor schließt eine Vielzahl von Sensorelementen (502) ein, die in einem Muster angeordnet sind, das eine verbesserte Möglichkeit der Feuchtigkeitserkennung bereitstellt, vorzugsweise mit mehreren Sensorelementen in Bereichen mit höherer Neigung zu variabler Feuchtigkeit oder Temperatur.

14. Ein Feuchtigkeitsüberwachungssystem gemäß Anspruch 13, wobei sich das eine oder die mehreren Sensorelemente über einen Rand davon ausdehnen und darüberhinaus eine Deckschicht (508) über den Sensorelementen einschließen, die Deckschicht erstreckt sich über einen Rand des absorbierenden Artikels (500) und schließt eine Vorrichtung für den Einsatz einer Signalempfängereinheit (506) ein, die an einem oder mehreren der Sensorelemente befestigt werden kann.

15. Ein Feuchtigkeitsüberwachungssystem gemäß Anspruch 13 oder Anspruch 14, das darüberhinaus eine Signalempfängereinheit (506) einschließt, die mit den Sensorelementen (502) verbunden werden kann, wobei die Signalempfängereinheit eine Speichervorrichtung für die Speicherung der Sensorsignale einschließt, die über einen bestimmten Zeitraum gesammelt wurden und vorzugsweise, wobei die Signalempfängereinheit eine Vorrichtung für den Empfang von Daten einschließt, die sich auf die Toilettengangaktivitäten eines Patienten beziehen.

16. Ein Feuchtigkeitsüberwachungssystem gemäß eines der Ansprüche 13 bis 15, wobei das Muster Sensorelemente (502) einschließt, die zu den Seiten des absorbierenden Artikels (500) positioniert sind, nahe einer Öffnung für die Aufnahme eines Beins des Trägers.

17. Ein Feuchtigkeitsüberwachungssystem gemäß eines der Ansprüche 13 bis 14, wobei der Sensor (502) ein Sensorsubstrat einschließt, das Sensorsubstrat hat einen oder mehrere Kanäle, die zwischen benachbarten Elementen des Sensors angeordnet sind, so dass bei der Verwendung in einem absorbierenden Artikel (500) mit darin angeordnetem superabsorbierendem Material, Flüssigkeit aus dem einen oder den mehreren Kanälen in das Sensorsubstrat gesaugt wird.

18. Ein Feuchtigkeitsüberwachungssystem gemäß eines der Ansprüche 13 bis 17, wobei die Sensorelemente Feuchtigkeit an verschiedenen identifizierbaren Stellen des absorbierenden Artikels erkennen, die Stellen sind dabei ausgewählt aus der Gruppe, die folgende Elemente einschließt:

   (a) zur Vorderseite des absorbierenden Artikels;
   (b) zur Rückseite des absorbierenden Artikels;
   (c) zur Seite des absorbierenden Artikels; und
   (d) im Wesentlichen zentral zum absorbierenden Artikel.

19. Ein Verfahren für die Feuchtigkeitsüberwachung in einem absorbierenden Artikel, das die folgenden Schritte einschließt:

(a) den Empfang (402) in einem Prozessor eines oder mehrerer Sensorsignale, die mit dem absorbierenden Artikel verbunden sind, wobei die Sensorsignale die Feuchtigkeit im absorbierenden Artikel anzeigen;
(b) die Verwendung des Prozessors, um die Sensorsignale unter Verwendung eines Algorithmus (404) zu analysieren; und
(c) die Charakterisierung eines Feuchtigkeitsereignisse im absorbierenden Artikel;
**gekennzeichnet dadurch, dass** der Algorithmus ein oder mehrere Sensorsignale auf ein vorbestimmtes mathematisches Modell anwendet, um ein Feuchtigkeitsereignis in einem absorbierenden Artikel zu charakterisieren und einen Vorhersagebereich, auf der Grundlage der aus den Sensorsignalen erkannten Muster, zu bestimmen, und
**gekennzeichnet dadurch, dass** Parameter des mathematischen Modells über eine Eingewöhnungszeit bestimmt werden, indem der Prozessor (i) kontinuierlich Sensorsignale empfängt, die die Feuchtigkeit anzeigen und bei jeder Änderung der Sensorwerte, Beobachtungsdaten empfängt, die (a) dem Wechsel des Polsters, (b) der Überprüfung des Polsters und (c) dem Wiegen des Polsters entsprechen, (ii) eine Regressionsanalyse ausführt, um die Parameter für das mathematische Modell zu gestalten; (iii) die Parameter zurück in das mathematische Modell speist, um ein Vertrauensniveau zu bestimmen, das anzeigt, wie genau das mathematische Modell die tatsächlichen Ereignisse, die von den Beobachtungsdaten (406) definiert sind, schätzt; und (iv) die Schritte (i) bis (iii) wiederholt, bis ein akzeptables Vertrauensniveau erreicht ist.

20. Ein Feuchtigkeitsüberwachungssystem gemäß Anspruch 19, wobei die Charakterisierung eines Feuchtigkeitsereignisses die Nachprüfung eines oder beider der folgenden Elemente einschließt:

(i) eine geschätzte Menge eines Exsudats in einem Feuchtigkeitsereignis; und
(ii) die Art des Exsudats.

21. Ein Verfahren für die Feuchtigkeitsüberwachung gemäß Anspruch 19 oder Anspruch 20, wobei das vorbestimmte mathematische Modell eine oder mehrere Variablen hat, die von dem einen oder den mehreren Sensorsignalen abgeleitet sind.

22. Ein Verfahren für die Feuchtigkeitsüberwachung gemäß eines der Ansprüche 19 bis 21, das darüberhinaus eines oder mehrere der folgenden Elemente einschließt:

(a) die Ableitung eines Toilettengang- oder Harnabgangsplan für ein Individuum, auf der Grundlage eines Feuchtigkeitsereignisses, das unter Verwendung des Systems überwacht wird;
(b) die Vorhersage, auf der Grundlage eines abgeleiteten Toilettengang- oder Harnabgangsplans, wann ein Individuum wahrscheinlich ein Feuchtigkeitsereignis erfährt, das die vorbestimmten Kriterien für eine manuelle Überprüfung erfüllt; und
(c) die Neukonfiguration des mathematischen Modells für die Verwendung bei einem oder mehreren bestimmten überwachten Individuen, einem unterschiedlichen Sensortyp und einem bestimmten Typ eines absorbierenden Artikels, durch:

(i) die Verwendung des bestimmten Individuums, des unterschiedlichen Sensortyps oder des bestimmten absorbierenden Artikeltyps für eine Eingewöhnungszeit, die Überwachung der Feuchtigkeit in regelmäßigen Abständen durch Erfassen der Sensorsignale und der Beobachtungsdaten; und
(ii) die Neukonfiguration des mathematischen Modells, so dass eine ausreichende Korrelation zwischen den Schätzungen, die unter Verwendung der Sensorsignale und des neu konfigurierten mathematischen Modells erzeugt wurden, und den Beobachtungsdaten, die während der Eingewöhnungszeit erfasst wurden, hergestellt wird.

23. Ein Verfahren für die Feuchtigkeitsüberwachung gemäß Anspruch 22, wobei die Neukonfiguration eines mathematischen Modells eines oder beide der folgenden Elemente einschließt:

(a) die Bestimmung neuer Parameter für das mathematische Modell; und
(b) die Anwendung eines linearen Regressionsalgorithmus.

24. Ein Verfahren für die Feuchtigkeitsüberwachung gemäß Anspruch 22 oder Anspruch 23, wobei die Beobachtungsdaten eines oder mehrere der folgenden Elemente einschließen:

(a) demographische Informationen; und
(b) Patienteninformationen.

**Revendications**

1. Un système de surveillance d'humidité destiné à la surveillance d'une humidité dans un ou plusieurs articles absorbants, le système comprenant :

   une entrée (104) destinée à la réception d'un ou de plusieurs signaux de capteur indicatifs d'une présence d'humidité dans un article absorbant,
   un processeur (106) destiné au traitement des un ou plusieurs signaux de capteur, et
   une interface utilisateur (108) destinée à une communication avec un utilisateur du système,
   où le processeur (106) est adapté de façon à exécuter un algorithme destiné à l'exécution d'une analyse des signaux, grâce à quoi l'algorithme applique les signaux de capteur reçus à un modèle mathématique prédéterminé de façon à caractériser un événement d'humidité dans un article absorbant et à déterminer une plage de prédictions en fonction de modèles identifiés à partir des signaux de capteur, et
   **caractérisé en ce que** des paramètres du modèle mathématique sont déterminés sur une période d'apprentissage par le processeur (i) qui reçoit en continu des signaux de capteur indiquant une humidité et, à chaque variation de valeurs de capteur, la réception de données d'observation correspondant à (a) un remplacement du tampon, (b) un examen du tampon et (c) une pesée du tampon, (ii) qui exécute une analyse de régression de façon à formuler des paramètres pour le modèle mathématique, (iii) qui renvoie les paramètres vers le modèle mathématique de façon à déterminer un niveau de confiance indiquant le degré de précision avec lequel le modèle mathématique estime les événements réels définis par les données d'observation, et (iv) qui répète les opérations (i) à (iii) jusqu'à ce qu'un niveau de confiance acceptable soit atteint.

2. Un système de surveillance d'humidité selon la Revendication 1 où le processeur est adapté de façon à exécuter un algorithme destiné à concevoir automatiquement un modèle mathématique de caractérisation d'un événement d'humidité dans un article absorbant et éventuellement, de façon à reconfigurer un ou plusieurs modèles mathématiques destinés à une utilisation avec un ou plusieurs éléments parmi un sujet donné qui est surveillé, un type de capteur différent et un type d'article absorbant différent, par l'utilisation d'un algorithme de façon à déterminer un ou plusieurs paramètres destinés au modèle mathématique et l'obtention de signaux de capteur et de données d'observation sur une période d'apprentissage au moyen du sujet donné qui est surveillé, d'un type de capteur différent ou d'un type d'article absorbant différent, et la reconfiguration du modèle mathématique de sorte qu'il existe une corrélation satisfaisante entre a) des estimations produites au moyen de signaux de capteur et du modèle mathématique, et b) les données d'observation obtenues au cours de la période d'apprentissage.

3. Un système de surveillance d'humidité selon la Revendication 1 ou 2, où l'algorithme applique des variables dérivées des un ou plusieurs signaux de capteur au modèle mathématique dans le but de caractériser un événement d'humidité.

4. Un système de surveillance d'humidité selon la Revendication 1 où la caractérisation d'un événement d'humidité dans un article absorbant comprend la détermination d'un ou de plusieurs éléments parmi :

   (i) un volume estimé d'exsudat dans un événement d'humidité, et
   (ii) la nature de l'exsudat dans un événement d'humidité.

5. Un système de surveillance d'humidité selon la Revendication 1 où l'algorithme applique des variables dérivées des un ou plusieurs signaux de capteur à un modèle mathématique prédéterminé de façon à caractériser un événement d'humidité.

6. Un système de surveillance d'humidité selon la Revendication 5 où les variables dérivées des signaux de capteur sont sélectionnées dans le groupe comprenant :

   (i) une zone sous une courbe de signal de capteur,
   (ii) une valeur de signal de capteur la plus élevée dans une période temporelle prédéterminée,
   (iii) une valeur maximale d'un bord d'attaque du signal de capteur,
   (iv) un taux de déclin de signal de capteur après un bord d'attaque,

(v) un volume estimé dans un événement d'humidité antérieur,

(vi) une heure d'apparition d'un événement d'humidité,

(vii) une heure de fin d'un événement d'humidité, et

(viii) une durée d'un événement d'humidité,

(ix) une heure du jour d'un événement d'humidité, et

(x) une durée écoulée depuis un dernier événement d'humidité.

7. Un système de surveillance d'humidité selon l'une quelconque des Revendications précédentes où le processeur est configuré de façon à déterminer une prédiction d'événement d'humidité comprenant un ou plusieurs éléments parmi :

(a) une probabilité d'un événement d'humidité imminent,

(b) une estimation du moment où un événement d'humidité est susceptible de se produire,

(c) une estimation d'un degré de saturation d'un article absorbant, et

(d) une estimation du moment où un article absorbant est susceptible d'atteindre sa capacité d'absorption.

8. Un système de surveillance d'humidité selon l'une quelconque des Revendications précédentes où le processeur est configuré de façon à fournir un ou plusieurs éléments parmi :

(a) un journal de propreté ou de miction,

(b) un échéancier de propreté ou de miction pour un sujet, en fonction d'événements d'humidité surveillés au moyen du système de surveillance d'humidité, par exemple sur un nombre de jours,

(c) une indication du moment où un sujet est susceptible de connaître un événement d'humidité qui répond à des critères prédéfinis pour une vérification manuelle en fonction d'un échéancier de propreté ou de miction dérivé,

(d) une alerte de communication générée et transmise automatiquement à un personnel soignant lorsqu'un ou plusieurs des critères prédéfinis pour une vérification manuelle sont satisfaits,

(e) une classification indiquant une forme possible d'incontinence subie par un patient surveillé par le système, la forme d'incontinence étant sélectionnée dans le groupe comprenant incontinence urinaire, fécale, de miction goutte à goutte, de stress, de débordement, par impériosité, urinaire mixte (MUI), totale et fonctionnelle, et

(f) une indication d'humidité persistante détectée ou prédite dans une zone d'un article absorbant.

9. Un système de surveillance d'humidité selon l'une quelconque des Revendications précédentes où le processeur est adapté de façon à recevoir des données de patient destinées à une utilisation avec le modèle mathématique, les données de patient comprenant une ou plusieurs données sélectionnées dans un groupe comprenant mais non limité à poids, taille, âge, sexe, conditions préexistantes, médications et opérations chirurgicales récentes.

10. Un système de surveillance d'humidité selon l'une quelconque des Revendications précédentes adapté de façon à reconfigurer un ou plusieurs modèles mathématiques destinés à une utilisation avec un ou plusieurs éléments parmi un sujet donné qui est surveillé, un type de capteur différent et un type d'article absorbant donné, par :

pendant une période d'apprentissage au moyen du sujet donné, du type de capteur différent ou du type d'article absorbant donné, la surveillance d'une humidité à des intervalles réguliers par l'obtention de signaux de capteur et l'obtention de données d'observation, et

la reconfiguration du modèle mathématique de sorte qu'il existe une corrélation satisfaisante entre les estimations produites au moyen des signaux de capteur et le modèle mathématique reconfiguré et les données d'observation obtenues au cours de la période d'apprentissage.

11. Un système de surveillance d'humidité selon la Revendication 10 où la reconfiguration d'un modèle mathématique implique un ou plusieurs éléments parmi :

(a) la détermination d'un ou de plusieurs nouveaux paramètres ou coefficients pour le modèle mathématique, et

(b) l'application d'un algorithme de régression linéaire.

12. Un système de surveillance d'humidité selon la Revendication 10 ou 11 où les données d'observation comprennent un ou plusieurs éléments parmi :

(a) des informations démographiques, et

(b) des informations de patient.

**13.** Un système de surveillance d'humidité selon l'une quelconque des Revendications précédentes, comprenant en outre un capteur (502) appliqué à ou incorporé dans un article absorbant (500) qui est surveillé en termes d'humidité au moyen du système de surveillance d'humidité, le capteur comprenant une pluralité d'éléments capteurs (502) agencés selon un motif qui fournit une capacité améliorée de détection d'humidité, de préférence avec plus d'éléments capteurs dans des zones possédant une propension plus élevée à une humidité ou température variable.

**14.** Un système de surveillance d'humidité selon la Revendication 13, où les un ou plusieurs éléments capteurs s'étendent au-delà d'un bord de celui-ci et comprenant en outre une couche de couverture (508) par dessus les éléments capteurs, la couche de couverture s'étendant au-delà d'un bord de l'article absorbant (500) et comprenant un moyen de logement d'une unité de réception de signaux (506) pouvant être fixée à un ou plusieurs des éléments capteurs.

**15.** Un système de surveillance d'humidité selon la Revendication 13 ou 14 comprenant en outre une unité de réception de signaux (506) pouvant être raccordée aux éléments capteurs (502), où l'unité de réception de signaux comprend un moyen à mémoire destiné à la conservation en mémoire de signaux de capteur recueillis sur une période de temps et de préférence où l'unité de réception de signaux comprend un moyen destinée à la réception de données relatives à des activités de propreté d'un patient.

**16.** Un système de surveillance d'humidité selon l'une quelconque des Revendications 13 à 15 où le motif comprend des éléments capteurs (502) positionnés vers les côtés de l'article absorbant (500), près d'une ouverture destinée à la réception d'une jambe du porteur.

**17.** Un système de surveillance d'humidité selon l'une quelconque des Revendications 13 à 14 où le capteur (502) comprend un substrat de capteur, le substrat de capteur possédant un ou plusieurs canaux agencés entre des éléments adjacents du capteur de sorte que, en utilisation, avec un article absorbant (500) possédant un matériau super absorbant agencé dans celui-ci, un fluide est aspiré à partir des un ou plusieurs canaux dans le substrat de capteur.

**18.** Un système de surveillance d'humidité selon l'une quelconque des Revendications 13 à 17 où les éléments capteurs détectent une humidité au niveau de divers emplacements identifiables par rapport à l'article absorbant, les emplacements étant sélectionnés dans le groupe comprenant :

(a) vers l'avant de l'article absorbant,
(b) vers l'arrière de l'article absorbant,
(c) vers un côté de l'article absorbant, et
(d) sensiblement au centre de l'article absorbant.

**19.** Un procédé de surveillance d'une humidité dans un article absorbant comprenant les opérations suivantes :

(a) la réception (402) au niveau d'un processeur d'un ou de plusieurs signaux de capteur associés à l'article absorbant, les signaux de capteur indiquant une humidité dans l'article absorbant,
(b) l'utilisation du processeur de façon à analyser les signaux de capteur au moyen d'un algorithme (404), et
(c) la caractérisation d'un événement d'humidité dans l'article absorbant, **caractérisé en ce que** l'algorithme applique un ou plusieurs signaux de capteur à un modèle mathématique prédéterminé de façon à caractériser un événement d'humidité dans un article absorbant et à déterminer une plage de prédictions en fonction de modèles identifiés à partir des signaux de capteur, et
**caractérisé en ce que** des paramètres du modèle mathématique sont déterminés sur une période d'apprentissage par le processeur (i) qui reçoit en continu des signaux de capteur indiquant une humidité et à chaque variation de valeurs de capteur, qui reçoit des données d'observation correspondant à (a) un remplacement du tampon, (b) un examen du tampon, et (c) une pesée du tampon, (ii) qui exécute une analyse de régression de façon à formuler des paramètres pour le modèle mathématique, (iii) qui renvoie des paramètres vers le modèle mathématique de façon à déterminer un niveau de confiance indiquant le degré de précision avec lequel le modèle mathématique estime les événements réels définis par les données d'observation (406), et (iv) qui répète les opérations (i) à (iii) jusqu'à ce qu'un niveau de confiance acceptable soit atteint.

**20.** Un procédé de surveillance d'une humidité selon la Revendication 19 où la caractérisation d'un événement d'humidité implique la vérification d'un élément ou les deux parmi :

(i) un volume estimé d'exsudat dans un événement d'humidité, et

(ii) la nature de l'exsudat.

**21.** Un procédé de surveillance d'une humidité selon la Revendication 19 ou 20 où le modèle mathématique prédéterminé possède une ou plusieurs variables dérivées des un ou plusieurs signaux de capteur.

**22.** Un procédé de surveillance d'une humidité selon l'une quelconque des Revendications 19 à 21 comprenant en outre une ou plusieurs opérations parmi :

(a) la dérivation d'un échéancier de propreté ou de miction pour un sujet, en fonction d'événements d'humidité surveillés au moyen du système,

(b) la prédiction, en fonction d'un échéancier de propreté ou de miction dérivé, du moment où un sujet est susceptible de connaître un événement d'humidité qui répond à des critères prédéfinis pour une vérification manuelle, et

(c) la reconfiguration du modèle mathématique destiné à une utilisation avec un ou plusieurs éléments parmi un sujet donné qui est surveillé, un type de capteur différent et un type d'article absorbant donné, par :

(i) pendant une période d'apprentissage au moyen du sujet donné, du type de capteur différent et/ou du type d'article absorbant donné, la surveillance d'une humidité à des intervalles réguliers par l'obtention de signaux de capteur et l'obtention de données d'observation, et

(ii) la reconfiguration du modèle mathématique de sorte qu'il existe une corrélation satisfaisante entre les estimations produites au moyen des signaux de capteur et du modèle mathématique reconfiguré et les données d'observation obtenues au cours de la période d'apprentissage.

**23.** Un procédé de surveillance d'une humidité selon la Revendication 22 où la reconfiguration d'un modèle mathématique implique une opération ou les deux parmi :

(a) la détermination de nouveaux paramètres pour le modèle mathématique, et

(b) l'application d'un algorithme de régression linéaire.

**24.** Un procédé de surveillance d'une humidité selon la Revendication 22 ou 23 où les données d'observation comprennent en outre un ou plusieurs éléments parmi :

(a) des informations démographiques, et

(b) des informations de patient.

FIG 1

FIG 2

FIG 3

FIG 4

FIG 5a

FIG 5b

FIG 6

FIG 7

FIG 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004100763 A
- US 2004220538 A
- CA 2361132
- US 2003011479 A
- US 5568128 A
- US 5416469 A
- WO 2005092177 A
- JP 2001318067 B
- JP 2002301098 B